(19) **Europäisches Patentamt / European Patent Office / Office européen des brevets**

(11) **EP 3 571 287 B1**

(12) # EUROPEAN PATENT SPECIFICATION

(45) Date of publication and mention
of the grant of the patent:
**27.12.2023 Bulletin 2023/52**

(21) Application number: **18702616.6**

(22) Date of filing: **12.01.2018**

(51) International Patent Classification (IPC):
**C12N 1/04** *(2006.01)*  **C12N 1/20** *(2006.01)*

(52) Cooperative Patent Classification (CPC):
**C12N 1/04; C12N 1/20**

(86) International application number:
**PCT/EP2018/050761**

(87) International publication number:
**WO 2018/134135 (26.07.2018 Gazette 2018/30)**

(54) **DRIED MICROORGANISM WITH EXCIPIENT**

GETROCKNETER MIKROORGANISMUS MIT HILFSSTOFF

MICRO-ORGANISME SÉCHÉ AYANT UN EXCIPIENT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **19.01.2017 US 201762448066 P
07.03.2017 EP 17159494**

(43) Date of publication of application:
**27.11.2019 Bulletin 2019/48**

(73) Proprietor: **International N&H Denmark ApS
2800 Kongens Lingby (DK)**

(72) Inventors:
• **HOLLARD, Christophe
Madison, WI 53716 (US)**
• **BABIN, Geoffrey
75017 Paris (FR)**

(74) Representative: **International N&H EMEA
Parallelvej 16
2800 Kongens Lyngby (DK)**

(56) References cited:
**WO-A1-2011/018509      WO-A1-2011/018547
WO-A2-2009/061222      CN-B- 103 431 318
US-A- 4 621 058**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Field of Invention

The present invention relates to the field of dried microorganisms.

### Background of the invention

It is common to dry microorganisms for storage, for example by freeze- or spray-drying. Such dried microorganisms are used in industrial and food uses, for example in the manufacture of cheese and yoghurt, and also as probiotics. During storage the viability of the dried organism degrades to some extent, so that with time the live cell count diminishes. This is particularly a problem in high-humidity environments.

Probiotics are live microorganisms which are beneficial for human or animal health when administered at appropriate dosages. One way of administering probiotics is through ingestion of dried probiotic mixed with excipients and packaged in capsules or sachets. Unfortunately, dried probiotic cells are not very stable during storage, with the result that live cell counts decrease with time, rendering the treatment less effective. This is particularly true when probiotics are blended with excipients having high humidity content, when container walls are permeable to external atmospheric moisture, and when the probiotic is stored in a high relative humidity environment.

### Summary of the invention

In a first aspect, the invention provides a composition comprising a blend of (a) dried microorganism, and (b) a phosphate salt in powder form; wherein the phosphate salt is selected from $K_2HPO_4$, $KH_2PO_4$, $Na_2HPO_4$, $NaH_2PO_4$, $MgHPO_4$, $Mg[H_2PO_4]_2$ and mixtures thereof; and wherein the phosphate salt is present in an amount of at least 50 wt% based on the total weight of microorganism and phosphate salt.

In a second aspect, the invention provides a unit microbial dose, containing a composition comprising a mixture of (a) dried microorganism, and (b) a phosphate salt in powder form; wherein the phosphate salt is selected from $K_2HPO_4$, $KH_2PO_4$, $Na_2HPO_4$, $NaH_2PO_4$, $MgHPO_4$, $Mg[H_2PO_4]_2$ and mixtures thereof; and wherein the phosphate salt is present in an amount of at least 50 wt% based on the total weight of microorganism and phosphate salt.

In a third aspect, the invention provides a process for the preparation of a composition comprising the steps:

(i) providing (a) a dried microorganism, and (b) a phosphate salt in powder form; and
(ii) mixing the dried microorganism, and the phosphate salt in powder form, to provide the composition

wherein the phosphate salt is selected from $K_2HPO_4$, $KH_2PO_4$, $Na_2HPO_4$, $NaH_2PO_4$, $MgHPO_4$, $Mg[H_2PO_4]_2$ and mixtures thereof; and wherein the phosphate salt is present in an amount of at least 50 wt% based on the total weight of microorganism and phosphate salt.

### Brief Description of the Drawings

**Figure 1** shows the % survival after 3 months' storage in humid conditions ($a_w$ = 0.4) at 30°C for different bacterial strains mixed with an excipient according to the invention ("K2HPO4") versus conventional excipient ("MCC") or no excipient ("Freeze-dried probiotic").
**Figure 2** shows the % survival after 3 months at 30°C as a function of $a_w$ ($a_w$ = 0.1, $a_w$ = 0.2 and $a_w$ = 0.3), for freeze-dried *Lactobacillus acidophilus* mixed with excipient according to the invention ("K2HPO4") versus conventional excipient ("MCC").
**Figure 3** shows the % survival of *Lactobacillus acidophilus* as a function of the percentage of $K_2HPO_4$ excipient in a blend under humid conditions ($a_w$ 0.4) at 30°C after 6 months.
**Figure 4** shows the % survival of *Lactobacillus acidophilus* as a function of the percentage of $K_2HPO_4$ excipient in a blend with MCC under humid conditions ($a_w$ 0.4) at 30°C after 6 months.
**Figure 5** shows the impact of pH of excipient on the % survival of freeze-dried *Lactobacillus acidophilus* powder under dry conditions ($a_w$ 0.1) at 30°C after 1 month and after 3 months. "KP" indicates $K_2HPO_4$ with pH adjusted to the indicated pH, "K2HPO4" indicates $K_2HPO_4$ without pH adjustment, and "MCC" indicates microcrystalline cellulose.
**Figure 6** shows the impact of pH of excipient on the % survival of freeze-dried *Lactobacillus acidophilus* powder under humid conditions ($a_w$ 0.4) at 30°C after 3 months. "K2HPO4" indicates $K_2HPO_4$ with pH adjusted to the indicated pH, "MCC" indicates microcrystalline cellulose.

## Detailed Description of the invention

The inventors have surprisingly found that the survival of dried microorganisms is improved by using a phosphate salt as excipient. The effect is particularly remarkable in high water activity ($a_w$) environments. A high water activity is considered to be $a_w > 0.15$.

Typically, microorganism dry powders, in particular probiotics, are blended with excipients to standardize the microorganism concentration. One of the most used excipients, especially when the microorganism is incorporated in capsules and sachets, is microcrystalline cellulose (MCC). The inventors have used MCC as a reference excipient. The inventors found that the use of phosphate salts can provide for greater than 60% survival of dried microorganism over 3 months compared to almost 0% survival in MCC excipient. This effect is particularly remarkable in high water activity environments ($a_w > 0.15$, particularly $a_w > 0.2$, more particularly $a_w > 0.3$).

Water activity is preferably measured by dew point hygrometer.

Microorganism survival rate is expressed in two different ways.

A) Survival percent

$$\% \text{ Survival} = (\text{CFU after storage} / \text{CFU } t_0) \times 100\%$$

B) Log loss

$$\text{Log loss} = \text{Log (CFU } t_0) - \text{Log (CFU after storage)}$$

## Dried Microorganism

The composition of the present invention contains a dried microorganism. The microorganism, in particular a probiotic, may be dried by any means, however, freeze-drying and spray-drying are preferred, with freeze-drying being particularly preferred.

The expression microorganism is meant to encompass any bacteria or yeast, or mixtures of these, and in particular a probiotic.

The term probiotic includes any live microorganisms which are administered to a host with a view to conferring a health benefit on the host. In particular, it may be a yeast or a bacterium, or mixtures of any of these.

The dried microorganism, in particular a probiotic, may be provided in any form suitable for delivery. For example, the dried microorganism, in particular a probiotic, may be provided in form of granules or powder. In one aspect the microorganism, in particular a probiotic, is in powder form.

The composition of the present invention may contain one species of microorganism, in particular a probiotic, one strain of microorganism, in particular a probiotic, a mixture of species of microorganism, in particular a probiotic, or a mixture of strains of microorganism, in particular a probiotic. In one aspect the composition of the present invention contains one species of microorganism, in particular a probiotic and, optionally, one strain of microorganism, in particular a probiotic. In one aspect the composition of the present invention contains a mixture of strains of microorganism, in particular a probiotic. In one aspect the composition of the present invention contains a mixture of species of microorganisms, in particular probiotics.

In a preferred embodiment, the microorganism, in particular a probiotic, is selected from lactobacilli, bifidobacteria, saccharomyces and mixtures thereof.

In a further preferred embodiment the microorganism, in particular a probiotic, is selected from species selected from *Bacillus coagulans, Bifidobacterium longum subsp. infantis, Lactobacillus acidophilus, Lactobacillus paracasei, Lactobacillus johnsonii, Lactobacillus plantarum, Lactobacillus reuteri, Lactobacillus reuteri protectis, Lactobacillus reuteri prodentis, Saccharomyces boulardii, Lactobacillus rhamnosus, Lactobacillus casei, Lactobacillus plantarum, Lactobacillus paracasei* and mixtures thereof.

In a further preferred embodiment the microorganism, in particular a probiotic, is selected from species selected from *Lactobacillus acidophilus, Lactobacillus casei, Bifidobacterium lactis,* and mixtures thereof.

In one aspect the microorganism, in particular a probiotic, is selected from microorganisms of the strains *Bacillus coagulans GBI-30, 6086, Bifidobacterium longum subsp. infantis 35624, Lactobacillus acidophilus NCFM, Lactobacillus paracasei St11 (or NCC2461), Lactobacillus johnsonii La1 (= Lactobacillus LC1, Lactobacillus johnsonii NCC533), Lactobacillus plantarum 299v, Lactobacillus reuteri ATCC 55730 (Lactobacillus reuteri SD2112), Lactobacillus reuteri protectis*

*(DSM 17938, daughter strain of ATCC 55730), Lactobacillus reuteri prodentis (DSM 17938/ATCC 55730 and ATCC PTA 5289 in combination), Saccharomyces boulardii, Lactobacillus rhamnosus GR-1, Lactobacillus reuteri RC-14, Lactobacillus acidophilus CL1285, Lactobacillus casei LBC80R, Lactobacillus plantarum HEAL 9, Lactobacillus paracasei 8700:2,* and mixtures thereof.

In one aspect the microorganism, in particular a probiotic, is selected from probiotics of the strains *Lactobacillus acidophilus (NCFM strain), Lactobacillus Casei (LPC37 strain), Bifidobacterium Lactis (HN0019)* and mixtures thereof.

The microorganism, in particular a probiotic, may be present in any suitable amount to deliver the required amount of microorganism, in particular a probiotic. The 'concentration' of the microorganism, in particular a probiotic in colony forming units (CFU) of microorganism per gram of the composition may also be selected by one skilled in the art. In one aspect the microorganism, in particular a probiotic is present in an amount of at least $1 \times 10^8$ CFU per gram of the composition. In one aspect the microorganism, in particular a probiotic is present in an amount of at least $1 \times 10^9$ CFU per gram of the composition. In one aspect the microorganism, in particular a probiotic is present in an amount of at least $1 \times 10^{10}$ CFU per gram of the composition. In one aspect the microorganism, in particular a probiotic, is present in an amount of from $1 \times 10^9$ to $5 \times 10^9$ CFU per gram of the composition.

When formulated as a unit probiotic dose, the dose contains any desired amount of probiotic. A typical unit dose will contain $10^8$ to $10^{14}$ CFU per dose, more preferably $10^9$ to $10^{12}$ CFU per dose, particularly preferably $10^9$ to $10^{11}$ CFU.

The microorganism, in particular a probiotic is in dried form, preferably spraydried or freeze-dried, in particular freeze-dried. Preferably the dried microorganism, in particular a probiotic has a water activity of no greater than 0.4, more preferably no greater than 0.3, particularly preferably no greater than 0.2. More particularly preferably, the dried microorganism, in particular a probiotic has a water activity of no greater than 0.1.

## Phosphate salt

The composition of the present invention contains a phosphate salt; wherein the phosphate salt is selected from $K_2HPO_4$, $KH_2PO_4$, $Na_2HPO_4$, $NaH_2PO_4$, $MgHPO_4$, $Mg[H_2PO_4]_2$ and mixtures thereof; and wherein the phosphate salt is present in an amount of at least 50 wt% based on the total weight of microorganism and phosphate salt. A phosphate salt is any salt of phosphoric acid ($H_3PO_4$), and includes salts based on dihydrogen phosphate ($H_2PO_4^-$), hydrogen phosphate ($HPO_4^{2-}$), and phosphate ($PO_4^{3-}$). In a preferred embodiment the composition of the present invention contains a salt of hydrogen phosphate ($HPO_4^{2-}$), or a mixture of hydrogen phosphate ($HPO_4^{2-}$) and dihydrogen phosphate ($H_2PO_4^-$).

Both hydrated and anhydrous phosphate salts can be used.

The cation of the phosphate salt is selected from sodium, potassium, and magnesium. Phosphate salts include $K_2HPO_4$, $KH_2PO_4$, $Na_2HPO_4$, $NaH_2PO_4$, $MgHPO_4$, $Mg[H_2PO_4]_2$, In a particularly preferred embodiment, the phosphate salt is a potassium phosphate salt.

Preferably the phosphate salt is dipotassium phosphate ($K_2HPO_4$), or a mixture of dipotassium phosphate ($K_2HPO_4$) and potassium dihydrogen phosphate ($KH_2PO_4$).

In a preferred embodiment, the phosphate salt is pH adjusted to between pH 6 and pH 9, preferably between pH 6.5 and 8, more particularly preferably pH 6.8 to 7.2. By the expression "pH of the salt" is meant the pH of a solution when the salt is dissolved in water. The pH may be adjusted by using blends of dihydrogen phosphate, hydrogen phosphate and phosphate. In a preferred embodiment a blend of $HPO_4^{2-}$ and $H_2PO_4^-$ salts is used. The relative amounts of these salts to yield a desired pH is well known. For example, a molar ratio of 61.5/38.5 $HPO_4^{2-}$/$H_2PO_4^-$ gives a pH of 7. Alternatively, pH adjusted salts may be prepared by titrating solutions of salts of $PO_4^{3-}$ and/or $HPO_4^{2-}$ with an acid or base to the desired pH and then drying the resulting solution, for example, by spray drying.

The phosphate salt is present in an amount of at least 50 wt% based on the total weight of microorganism and phosphate salt. The phosphate salt is present in the composition of the invention in an amount of at least 50 wt% based on the total weight of microorganism and phosphate salt to provide the desired stabilisation of the dried microorganism, in particular a probiotic. In the present context, the wt%'s are given with respect to the total weight of microorganism, in particular a probiotic and phosphate salt. The phosphate salt is present in an amount of at least 50% by weight of the composition. In one aspect, the phosphate salt is present in an amount of at least 60% by weight of the composition. In one aspect, the phosphate salt is present in an amount of at least 70% by weight of the composition. In one aspect, the phosphate salt is present in an amount of at least 80% by weight of the composition.

In one aspect, the phosphate salt is present in an amount of from 50 to 90% by weight of the composition. In one aspect, the phosphate salt is present in an amount of from 50 to 80 % by weight. In one aspect, the phosphate salt is present in an amount of from 50 to 90% by weight of the composition. In one aspect, the phosphate salt is present in an amount of from 60 to 90% by weight of the composition. In one aspect, the phosphate salt is present in an amount of from 70 to 90% by weight of the composition. In one aspect, the phosphate salt is present in an amount of from 80 to 90% by weight of the composition. In the present context, the wt%'s are given with respect to the total weight of microorganism, in particular a probiotic and phosphate salt.

When components other than microorganism, in particular a probiotic, and phosphate salt are present in the composition, the phosphate salt is present in an amount of at least 50% by weight of the total composition. In one aspect, the phosphate salt is present in an amount of at least 59%, or 60% by weight of the total composition. In one aspect, the phosphate salt is present in an amount of at least 70% by weight of the total composition. In one aspect, the phosphate salt is present in an amount of at least 80% by weight of the total composition.

A suitable mixture is 80 wt% phosphate salt and 20 wt% microorganism, in particular a probiotic.

The phosphate salt is preferably in the form of a powder. Preferably the particle size distribution has a D10 value in microns of 5-120 (more preferably 5-90), a D50 value in microns of 70-180 (more preferably 80-140), and a D90 value in microns of 160-400 (more preferably 180-350).

The survival rate of microorganism, in particular a probiotic is particularly increased under high water activity ($a_w > 0.15$, preferably $a_w > 0.2$, more preferably $a_w > 0.3$) conditions.

The composition of the invention preferably increases the survival of microorganism, in particular a probiotic by at least 30% as compared to microorganism alone, more preferably by at least 40%, particularly preferably more than 60%.

The composition of the invention preferably increases the survival of microorganism, in particular a probiotic under humid conditions ($a_w > 0.15$, preferably $> 0.2$, more preferably $> 0.3$) by at least 1% to 30% as compared to microorganism alone, more preferably by at least 20%, particularly preferably more than 30%.

The composition of the invention preferably increases the survival of probiotic by at least 30% as compared to probiotic with MCC as excipient, more preferably by at least 40%, particularly preferably more than 60%.

The composition of the invention preferably increases the survival of probiotic under humid conditions ($a_w > 0.15$, preferably $> 0.2$, more preferably $> 0.3$) by at least 30% as compared to probiotic with MCC as excipient, more preferably by at least 40%, particularly preferably more than 60%.

## Additional Components

The composition of the present invention may contain only probiotic and phosphate salt or it may contain one or more additional components.

In one embodiment the composition further comprises additional excipients such as maltodextrin, microcrystalline cellulose (MCC), prebiotics such as inulin, fructooligosaccharides, galactooligosaccharides, polydextrose, flow aid agents such silica, magnesium stearate.

When additional components are present, preferably they constitute less than 20 wt% of the total composition, more preferably less than 10 wt%.

## Use

The composition of the invention may be used for administration to a human or animal as a probiotic, or it may be used for industrial or food applications. Typical food applications include the production of cheese, yoghourt, fermented soy products (such as miso, natto, etc.), sauerkraut, comestible alcohol products, etc. Typical industrial applications include the production of raw or finished materials by fermentation such as industrial alcohol production.

## Forms

The composition of the invention may be in the form of a bulk powder mix, for example for storage or transport before food or industrial use or before administration to a human or animal, and/or before division into suitable dosage forms.

In a further aspect, the invention provides a unit microbial dose for administration to a human or animal. The unit microbial dose comprises a suitable amount of the composition of the invention, which may be packaged, for example, in sachets, capsules or tablets. A typical unit dose will contain $10^8$ to $10^{14}$ CFU per dose, more preferably $10^{10}$ to $10^{12}$ CFU per dose.

## Process

In a further aspect the invention provides a process for the preparation of the composition of the invention comprising (i) providing (a) a dried microorganism, and (b) a phosphate salt in powder form; (ii) mixing the dried microorganism, and the phosphate salt in powder form, to provide the composition wherein the phosphate salt is selected from $K_2HPO_4$, $KH_2PO_4$, $Na_2HPO_4$, $NaH_2PO_4$, $MgHPO_4$, $Mg[H_2PO_4]_2$ and mixtures thereof; and wherein the phosphate salt is present in an amount of at least 50 wt% based on the total weight of microorganism and phosphate salt. In a preferred embodiment of the process, the microorganism is freeze-dried.

The mixing can be performed by any method that does not damage the microorganism. For example, rotation or shaking in a suitable container, and/or mixing with a mixing implement, such as a paddle.

## EXAMPLES

### Materials

The experiments were conducted with the following freeze-dried probiotics: *Lactobacillus acidophilus* (NCFM strain), *Lactobacillus casei* (LPC37 strain), *Bifidobacterium lactis* (BBi), *Bifidobacterium lactis* (BBL), and *Bifidobacterium lactis* (HN0019).

Microcrystalline cellulose was supplied by Mingtai Chemical Company, and the di-potassium phosphate was obtained from BK Glulhi Gmbh Company.

Capsules used were Vcaps, size 0, CS, hypromellose from Capsugel cie.

Silica was Sipernat 50s, obtained from Evonik industries AG.

Magnesium stearate was obtained from Aceto corporation.

Maltodextrin (IT6) was obtained from Roquette.

### Analytical Methods

Water activity measurement ($a_w$)

An Aqualab 3TE, Decagon was used for the measurements of water activity. The sample (about 1g) is equilibrated within the headspace of a sealed chamber containing a mirror, an optical sensor, an internal fan and infrared temperature sensor.

### Cell count method

The cell count method used is the method from quality control laboratory according to the strain. The results were given in colony forming unit per gram of product (CFU/g).

The method consisted of:

(i) 1g of sample was weighed into a bottle; sterile peptone water was added up to 100g and the mixture was mixed for 5 minutes at 400 rpm using a bench top shaker. The mixture was left for 20 minutes at room temperature and then mixed again for 5 minutes to obtain a homogenous solution. A 10-2 dilution from the original sample was obtained.

(ii) Subsequent dilutions were carried out at 1:10 steps and were made by adding 1 ml of the solution to 9 ml of peptone water. The solutions were homogenized at each step for 20 seconds using a Vortex system at maximum speed.

(iii) MRS agar and 1% of cysteine was used to plate cells.

(iv) For each determination, 4 plates were counted: two different volumes of cell suspension were plated and each volume was made in duplicate.

Then the number of colonies obtained on the plates were added and divided by the total sum of the volumes of cell suspension used for these plates.

(vi) The plates were incubated at 37°C for 72 hours.

### Example 1

### Preparation of samples

The various probiotic species were blended with MCC to obtain a CFU between $1.5 \times 10^{10}$ and $3 \times 10^{10}$ CFU/g. The blends were mixed by rotation (about 60tr/min) in a plastic bottle for 20 min. Then, the capsules were filled with the blends. The preparation of the samples was made in a clean room at 40% RH and 25 deg. C.

### High humidity exposure tests

Maltodextrin was exposed to an atmosphere at 40% RH until equilibrium was reached. As a consequence, the $a_w$ of the maltodextrin was close to 0.4. The same method was used to obtain maltodextrin equilibrated at $a_w$'s of 0.3 and 0.1.

Capsules were prepared and filled with:

1. probiotic powder only,
2. probiotic (20 wt%)-MCC (80 wt%) blend, and
3. probiotic (20 wt%)-$K_2HPO_4$ (80 wt%) blend

These capsules were introduced into a glass bottle. The maltodextrin at an $a_w$ of approximately 0.4 was then added on top and the bottle was shaken for the desired time period.

The capsules were stored in an environmental chamber at 30°C for 6 months. CFU and $a_w$ were measured at time 0 months, 1 months, and 3 months to evaluate the impact of excipient type on stability performance.

**Calculation of survival**

Probiotic survival rate was expressed in two different ways.

A) Survival percent

$$\% \text{ Survival} = (\text{CFU after storage} / \text{CFU } t_0) \times 100\%$$

B) Log loss

$$\text{Log loss} = \text{Log (CFU } t_0) - \text{Log (CFU after storage)}$$

**Results**

Percent survival of different strains of bacteria in humid conditions ($a_w$ 0.4) after three months according to excipient used are shown in Table 1. MCC represents the case where the probiotic was mixed with MCC, $K_2HPO_4$ represents the case where the probiotics were mixed with $K_2HPO_4$, and "freeze-dried probiotic" represents the case where the probiotic powder alone was used.

**Table 1. Percent survival of different bacterial strains after three-months storage in humid conditions ($a_w$ 0.4) and dependence on excipient**

|  | *Lactobacillus acidophilus* | *Lactobacillus casei* | *Bifidobacterium lactis (BBi)* | *Bifidobacterium lactis (BBL)* | *Bifidobacterium lactis (HN019)* |
|---|---|---|---|---|---|
| MCC | 0% | 0% | 0% | 1% | 2% |
| $K_2HPO_4$ | 35% | 1% | 5% | 26% | 20% |
| Freeze-dried probiotic | 0% | 0% | 0% | 0% | 0% |

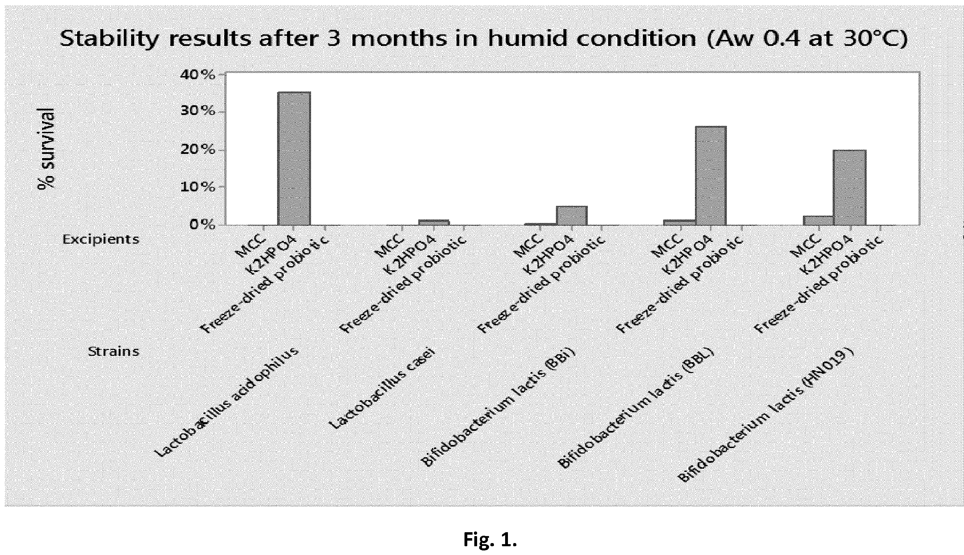

Figure 1 shows the same results in graphic form. MCC represents the case where the probiotic was mixed with MCC, $K_2HPO_4$ represents the case where the probiotics were mixed with $K_2HPO_4$, and "freeze-dried probiotic" represents the case where the probiotic powder alone was used.

Table 1 and Figure 1 show that the impact of $K_2HPO_4$ on stability varies as a function of strain. Survival is always higher with $K_2HPO_4$ as excipient than with MCC or probiotic alone. It is clear that the use of $K_2HPO_4$ as excipient improves the stability (survival) of the strains, and in particular in humid conditions.

*Lactobacillus acidophilus* survival under different humidity conditions

Percent survival of *Lactobacillus acidophilus* after three months at 30°C as a function of $a_w$ and excipient is shown in Table 2. MCC represents the case where the probiotic was mixed with MCC, $K_2HPO_4$ represents the case where the probiotics were mixed with $K_2HPO_4$.

| Table 2. Percent survival of *Lactobacillus acidophilus* after three months at 30°C and different $a_w$ and dependence on excipient | | | |
|---|---|---|---|
| $a_w$ | 0.1 | 0.3 | 0.4 |
| MCC | 78% | 6% | 0% |
| $K_2HPO_4$ | 66% | 39% | 60% |

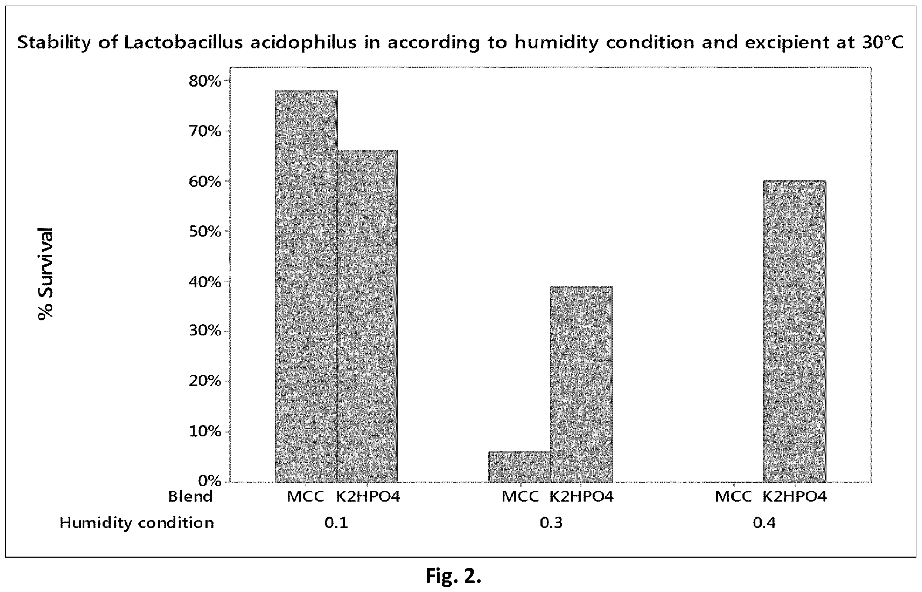

Figure 2 shows the same results in graphic form. MCC represents the case where the probiotic was mixed with MCC, $K_2HPO_4$ represents the case where the probiotics were mixed with $K_2HPO_4$.
Table 2 and Figure 2 show that the percent survival with $K_2HPO_4$ are always higher than the percent survival with MCC in the higher humidity conditions ($a_w$ > 0.1). In the lower humidity condition ($a_w$ = 0.1), the stabilities with $K_2HPO_4$ and MCC are similar.
From these results it can be seen that the addition of $K_2HPO_4$ improves the stability of strains in humid conditions.

**Example 2**

This example looks at stability of probiotics in a humid environment as a function of $K_2HPO_4$ concentration. Sipernat 50s and magnesium stearate were added as flow aids to have a sample composition similar to a commercial blend composition. For this example, freeze-dried *Lactobacillus acidophilus* powder was used. The example was prepared in two steps. In Part 1, the probiotic was mixed with $K_2HPO_4$ only. In Part 2, the probiotic was mixed with $K_2HPO_4$ and MCC.

**Preparation of samples**

**Part 1: Probiotics mixed with $K_2HPO_4$**

Blends consisting of freeze-dried *Lactobacillus acidophilus* powder, $K_2HPO_4$, Sipernat 50s, and Magnesium Stearate were prepared. Ten samples were made in which the concentration of freeze-dried *Lactobacillus acidophilus* was gradually increased and the concentration of $K_2HPO_4$ gradually decreased while the concentrations of Sipernat 50s and Stearate Magnesium were kept constant. The table below shows the description of the different samples for Part 1.

**Table 3: Description of different samples for Example 2, Part 1**

| | Sample 1 | Sample 2 | Sample 3 | Sample 4 | Sample 5 | Sample 6 | Sample 7 | Sample 8 | Sample 9 | Sample 10 |
|---|---|---|---|---|---|---|---|---|---|---|
| *Lactobacillus Acidophilus* (g) | 5 | 10 | 15 | 20 | 25 | 30 | 35 | 40 | 45 | 49.5 |
| $K_2HPO_4$ (g) | 44.5 | 39.5 | 34.5 | 29.5 | 24.5 | 19.5 | 14.5 | 9.5 | 4.5 | 0 |
| Sipernat 50s (g) | 0.25 | 0.25 | 0.25 | 0.25 | 0.25 | 0.25 | 0.25 | 0.25 | 0.25 | 0.25 |
| Magnesium stearate (g) | 0.25 | 0.25 | 0.25 | 0.25 | 0.25 | 0.25 | 0.25 | 0.25 | 0.25 | 0.25 |
| Total mass (g) | 50 | 50 | 50 | 50 | 50 | 50 | 50 | 50 | 50 | 50 |
| Cell count blend (CFU/g) | 3.5E10 | 7E10 | 1.05E11 | 1.4E11 | 1.75E11 | 2.1E11 | 2.45E11 | 2.8E11 | 3.15E11 | 3.47E11 |
| % $K_2HPO_4$ (W/W) | 89% | 79% | 69% | 59% | 49% | 39% | 29% | 19% | 9% | 0% |

**Part 2: Probiotics mixed with $K_2HPO_4$ and MCC**

Blends consisting of freeze-dried *Lactobacillus acidophilus* powder, and $K_2HPO_4$, MCC, Sipernat 50s, and Magnesium Stearate were prepared. Nine samples were made in which the concentration of $K_2HPO_4$ was gradually increased and the concentration of MCC gradually decreased, while the concentrations of the *Lactobacillus acidophilus* powder, Sipernat 50s and Stearate Magnesium were kept constant. The table below shows the description of the different samples for Part 2.

| Table 4: Description of different samples for Example 2, Part 2 | | | | | | | | | |
|---|---|---|---|---|---|---|---|---|---|
| | Sample 11 | Sample 12 | Sample 13 | Sample 14 | Sample 15 | Sample 16 | Sample 17 | Sample 18 | Sample 19 |
| *Lactobacillus Acidophilus* (g) | 7.5 | 7.5 | 7.5 | 7.5 | 7.5 | 7.5 | 7.5 | 7.5 | 7.5 |
| MCC (g) | 42 | 37 | 32 | 27 | 22 | 17 | 12 | 7 | 0 |
| $K_2HPO_4$ (g) | 0 | 5 | 10 | 15 | 20 | 25 | 30 | 35 | 42 |
| Sipernat 50s (g) | 0.25 | 0.25 | 0.25 | 0.25 | 0.25 | 0.25 | 0.25 | 0.25 | 0.25 |
| Magnesium stearate (g) | 0.25 | 0.25 | 0.25 | 0.25 | 0.25 | 0.25 | 0.25 | 0.25 | 0.25 |
| Total mass (g) | 50 | 50 | 50 | 50 | 50 | 50 | 50 | 50 | 50 |
| Cell count blend (CFU/g) | 5.25E10 | 5.25E10 | 5.25E10 | 5.25E10 | 5.25E10 | 5.25E10 | 5.25E10 | 5.25E10 | 5.25E10 |
| % K2HPO4 (W/W) | 0% | 10% | 20% | 30% | 40% | 50% | 60% | 70% | 84% |

**Results**

**Results of Part 1**

Percent survival of *Lactobacillus acidophilus* as a function of the weight percent of $K_2HPO_4$ excipient under humid conditions ($a_w$ 0.4) after six months at 30°C is shown in Table 5.

| Table 5. Percent survival of *Lactobacillus acidophilus* as a function of the percent of $K_2HPO_4$ excipient under humid conditions ($a_w$ 0.4) after six months at 30°C ||
|---|---|
| wt% $K_2HPO_4$ in blend | % Survival |
| 0% | 0% |
| 9% | 0% |
| 19% | 0% |
| 29% | 0% |
| 39% | 0% |
| 49% | 0% |
| 59% | 2% |
| 69% | 9% |
| 79% | 17% |
| 89% | 12% |

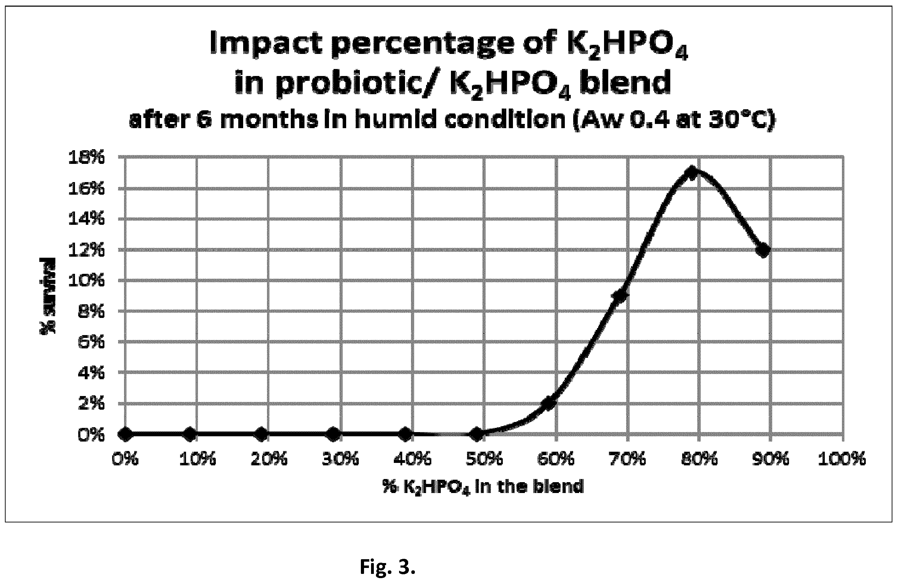

Figure 3 shows the same results in graphic form.
Table 5 and Figure 3 show that the survival varies as a function of the amount of $K_2HPO_4$. The results show that above 50 wt% $K_2HPO_4$ the survival is significantly increased. Above 80 wt% $K_2HPO_4$ survival is still significantly better than without $K_2HPO_4$, although less than at 80 wt%.

**Results of Part 2**

Percent survival of *Lactobacillus acidophilus* as a function of the percentage of $K_2HPO_4$ excipient in a blend with MCC under humid conditions ($a_w$ 0.4) at 30°C after 6 months are shown in Table 6.

| Table 6. Percent survival of *Lactobacillus acidophilus* as a function of the percentage of $K_2HPO_4$ excipient in a blend with MCC under humid conditions ($a_w$ 0.4) at 30°C after 6 months ||
|---|---|
| % K2HPO4 in blend | %Survival |
| 0% | 0.0% |
| 0% | 0.0% |
| 10% | 0.0% |
| 20% | 0.0% |
| 30% | 0.0% |
| 40% | 0.2% |
| 50% | 0.8% |
| 60% | 3.2% |
| 70% | 15.3% |
| 84% | 20.4% |

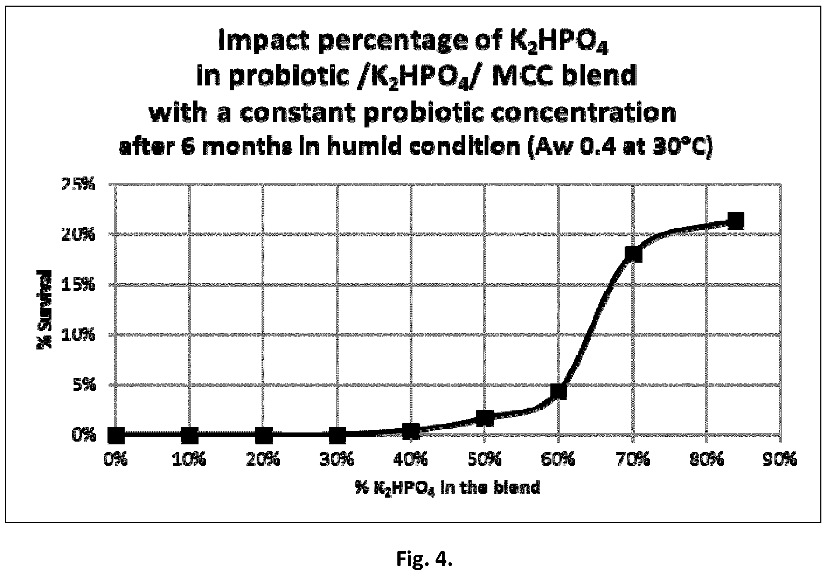

Figure 4 shows the same results in graphic form.

Table 6 and Figure 4 show that the survival varies as a function of the $K_2HPO_4$ percentage, and above 40 wt% $K_2HPO_4$ is significantly better than without $K_2HPO_4$.

**Example 3**

The effect of pH of $K_2HPO_4$ excipient on *Lactobacillus acidophilus, Bifidobacterium lactis, Lactobacillus casei* was evaluated.

Different excipients comprising $K_2HPO_4$ with various pH values were made (pH 6.5; pH 6.9; pH 7.1; pH 7.6 pH 8.1 and pH 9). The excipients at different pH's were prepared by either of two methods:

1. A solution of $K_2HPO_4$ was prepared and phosphoric acid added to bring the pH of the solution to the desired value, and the solution was then spray dried to yield a powder; or
2. $K_2HPO_4$ was blended in dry form with various amounts of $KH_2PO_4$ calculated to give the desired pH.

**Preparation of samples**

**Part 1: Impact of pH of excipient on survival under dry conditions:**

Freeze-dried *Lactobacillus acidophilus* powder was blended with the different excipients at various pH's. The composition of each blend was 80% of excipient $K_2HPO_4$ powder and 20% freeze-dried probiotic powder. The blends were mixed by rotation (about 60tr/min) in plastic bottles for 20 min. Sachets were filled with the blends. The preparation of the samples was made in a clean room at 40% RH and 25°C. During testing the sachets were stored at 30°C in dry humidity ($a_w \leq 0.1$) for 3 months.

**Part 2: Impact of pH of excipient on survival under humid conditions:**

Freeze-dried *Lactobacillus acidophilus* powder was blended with the different $K_2HPO_4$ powders at different pH's and MCC. The composition of each blend was 80% of excipient and 20% of freeze dry probiotic powder. The blends were mixed by rotation (about 60tr/min) in plastic bottles for 20 min. The preparation of the samples was made in a clean room at 40% RH and 25°C. Capsules were filled with the blends and the capsules were introduced into a glass bottle. Maltodextrin at an $a_w$ of approximately 0.4 was then added on top and the bottle was shaken. The bottles were stored at 30°C for 3 months.

A control with 80% of dry MCC and 20% of freeze-dried probiotic powder was prepared and tested in the same way.

**Results**

**Results of part 1**

Survival of freeze-dried *Lactobacillus acidophilus* as a function of pH of $K_2HPO_4$ excipient after storage under dry conditions ($a_w \leq 0.1$) at 30°C for one and three months is shown in Table 7.

| Table 7. Survival of freeze-dried *Lactobacillus acidophilus* as a function of pH of $K_2HPO_4$ excipient after storage under dry conditions ($a_w \leq 0.1$) at 30°C for one and three months | | |
|---|---|---|
| Excipient | % Survival after 1 month | % Survival after 3 months |
| $K_2HPO_4$ pH 6.5 | 63% | 59% |
| $K_2HPO_4$ pH 6.9 | 87% | 88% |
| $K_2HPO_4$ pH 7.1 | 73% | 63% |
| $K_2HPO_4$ pH 7.6 | 68% | 49% |
| $K_2HPO_4$ pH 8.1 | 71% | 68% |
| $K_2HPO_4$ (pH unadjusted) | 58% | 59% |
| MCC | 50% | 12% |

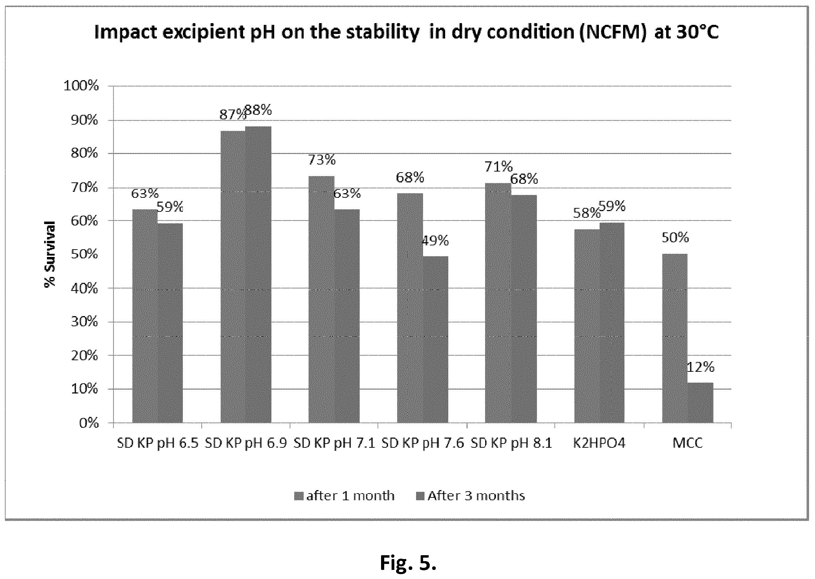

Figure 5 shows the same results in graphic form.

Table 7 and Figure 5 show that survival/stability under dry conditions varies as a function of pH, but in all cases is better than survival with MCC as sole excipient. Stability was impacted by the pH of excipient. For stability under dry conditions, the optimal pH of $K_2HPO_4$ is between 6.9 and 7.1. Other species evaluated included *Bifidobacterium lactis* and *Lactobacillus casei.*

**Results of Part 2**

Survival of freeze-dried Lactobacillus acidophilus as a function of pH of $K_2HPO_4$ excipient after storage under humid conditions ($a_w = 0.4$) at 30°C for three months are shown in Table 8.

| Table 8. Survival of freeze-dried *Lactobacillus acidophilus* as a function of pH of $K_2HPO_4$ excipient after storage under humid conditions ($a_w = 0.4$) at 30°C for three months | |
|---|---|
| Excipient | % Survival |
| $K_2HPO_4$ pH 6.5 | 37% |
| $K_2HPO_4$ pH 6.9 | 50% |
| $K_2HPO_4$ pH 7.1 | 13% |
| $K_2HPO_4$ pH 7.6 | 14% |
| $K_2HPO_4$ pH 8.1 | 26% |
| $K_2HPO_4$ pH 9 | 41% |
| MCC | 1% |

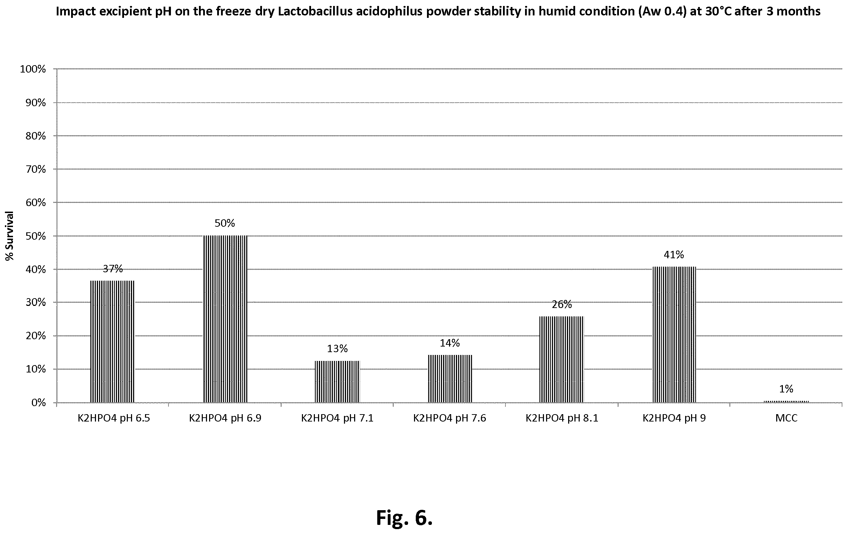

Figure 6 shows the same results in graphic form.

Table 8 and Figure 6 show that the stability varies as function of pH value. All excipients with pH adjusted have a better stability than MCC powder under humid conditions. To obtain the best stability under humid conditions, the optimal pH is 6.9.

**Claims**

1. A composition comprising a blend of (a) dried microorganism, and (b) a phosphate salt in powder form, wherein the phosphate salt is selected from $K_2HPO_4$, $KH_2PO_4$, $Na_2HPO_4$, $NaH_2PO_4$, $MgHPO_4$, $Mg[H_2PO_4]_2$ and mixtures thereof, and wherein the phosphate salt is present in an amount of at least 50 wt% based on the total weight of microorganism and phosphate salt.

2. The composition of claim 1, wherein the phosphate salt is selected from $K_2HPO_4$, $KH_2PO_4$, $Na_2HPO_4$, $NaH_2PO_4$, and mixtures thereof.

3. The composition of claim 1, wherein the phosphate salt is selected from $K_2HPO_4$, $KH_2PO_4$, and mixtures thereof.

4. The composition of claim 1, 2 or 3, wherein the phosphate salt is a mixture of salts of $HPO_4^{2-}$ and $H_2PO_4^-$.

5. The composition of any one preceding claim, wherein the phosphate salt is pH adjusted to between pH 6 and 9.

6. The composition of any one preceding claims, wherein the phosphate salt is pH adjusted to between 6.8-7.2.

7. The composition of any one preceding claim, wherein the phosphate salt is present in an amount of at least 60 wt% based on the total weight of microorganism and phosphate salt.

8. The composition of any one preceding claim, wherein the phosphate salt is present in an amount of at least 80 wt% based on the total weight of microorganism and phosphate salt.

9. The composition of any one preceding claim, wherein the microorganism is freeze-dried.

10. The composition of any one preceding claim, wherein the microorganism is a probiotic.

11. The composition of any one preceding claim, wherein the microorganism is selected from lactobacilli, bifidobacteria, saccharomyces and mixtures thereof.

12. The composition of any one preceding claim, wherein the microorganism is selected from *Bacillus coagulans, Bifidobacterium longum subsp. infantis, Lactobacillus acidophilus, Lactobacillus paracasei, Lactobacillus johnsonii, Lactobacillus plantarum, Lactobacillus reuteri, Lactobacillus reuteri protectis, Lactobacillus reuteri prodentis, Saccharomyces boulardii, Lactobacillus rhamnosus, Lactobacillus casei, Lactobacillus plantarum, Lactobacillus paracasei* and mixtures thereof.

13. The composition of any one preceding claim, wherein the microorganism is a *Lactobacillus acidophilus.*

14. A unit microbial dose comprising a composition according to any one of claims 1 to 13.

15. The unit microbial dose of claim 14, in the form of a capsule containing the composition.

16. The unit microbial dose of claim 14, in the form of a sachet containing the composition.

17. The unit microbial dose of claim 14, in the form of a tablet.

18. The unit microbial dose of claim 14, in the form of a powdered nutritional formula.

19. A process for the preparation of the composition of any one of claims 1 to 13, comprising the steps of:

(i) providing (a) a dried microorganism, and (b) a phosphate salt in powder form; and
(ii) mixing the dried microorganism, and the phosphate salt in powder form, to provide the composition,

wherein the phosphate salt is selected from $K_2HPO_4$, $KH_2PO_4$, $Na_2HPO_4$, $NaH_2PO_4$, $MgHPO_4$, $Mg[H_2PO_4]_2$ and mixtures thereof; and wherein the phosphate salt is present in an amount of at least 50 wt% based on the total weight of microorganism and phosphate salt.

**Patentansprüche**

1. Zusammensetzung, die eine gleichförmige Mischung aus (a) getrocknetem Mikroorganismus und (b) einem Phosphatsalz in Pulverform umfasst, wobei das Phosphatsalz aus $K_2HPO_4$, $KH_2PO_4$, $Na_2HPO_4$, $NaH_2PO_4$, $MgHPO_4$, $Mg[H_2PO_4]_2$ und deren Mischungen ausgewählt ist, und wobei das Phosphatsalz in einer Menge von mindestens 50 Gew.-% vorliegt, bezogen auf das Gesamtgewicht an Mikroorganismus und Phosphatsalz.

2. Zusammensetzung nach Anspruch 1, wobei das Phosphatsalz aus K2HPO4, KH2PO4, Na2HPO4, NaH2PO4 und deren Mischungen ausgewählt ist.

3. Zusammensetzung nach Anspruch 1, wobei das Phosphatsalz aus K2HPO4, KH2PO4 und deren Mischungen ausgewählt ist.

4. Zusammensetzung nach Anspruch 1, 2 oder 3, wobei es sich bei dem Phosphatsalz um eine Mischung aus Salzen von HPO42- und H2PO4- handelt.

5. Zusammensetzung nach einem beliebigen vorhergehenden Anspruch, wobei das Phosphatsalz eine pH-Einstellung auf einen pH-Wert zwischen 6 und 9 erfährt.

6. Zusammensetzung nach einem beliebigen vorhergehenden Anspruch, wobei das Phosphatsalz eine pH-Einstellung auf 6,8 bis 7,2 erfährt.

7. Zusammensetzung nach einem beliebigen vorhergehenden Anspruch, wobei das Phosphatsalz in einer Menge von mindestens 60 Gew.-% vorliegt, bezogen auf das Gesamtgewicht an Mikroorganismus und Phosphatsalz.

8. Zusammensetzung nach einem beliebigen vorhergehenden Anspruch, wobei das Phosphatsalz in einer Menge von mindestens 80 Gew.-% vorliegt, bezogen auf das Gesamtgewicht an Mikroorganismus und Phosphatsalz.

9. Zusammensetzung nach einem beliebigen vorhergehenden Anspruch, wobei der Mikroorganismus gefriergetrocknet ist.

10. Zusammensetzung nach einem beliebigen vorhergehenden Anspruch, wobei es sich bei dem Mikroorganismus um ein Probiotikum handelt.

11. Zusammensetzung nach einem beliebigen vorhergehenden Anspruch, wobei der Mikroorganismus aus Lactobacilli, Bifidobacteria, Saccharomyces und deren Mischungen ausgewählt ist.

12. Zusammensetzung nach einem beliebigen vorhergehenden Anspruch, wobei der Mikroorganismus aus Bacillus coagulans, Bifidobacterium longum subsp. infantis, Lactobacillus acidophilus, Lactobacillus paracasei, Lactobacillus johnsonii, Lactobacillus plantarum, Lactobacillus reuteri, Lactobacillus reuteri protectis, Lactobacillus reuteri prodentis, Saccharomyces boulardii, Lactobacillus rhamnosus, Lactobacillus casei, Lactobacillus plantarum, Lactobacillus paracasei und deren Mischungen ausgewählt ist.

13. Zusammensetzung nach einem beliebigen vorhergehenden Anspruch, wobei es sich bei dem Mikroorganismus um einen Lactobacillus acidophilus handelt.

14. Mikrobielle Dosiseinheit, die eine Zusammensetzung gemäß einem beliebigen der Ansprüche 1 bis 13 umfasst.

15. Mikrobielle Dosiseinheit nach Anspruch 14, in Form einer Kapsel, welche die Zusammensetzung enthält.

16. Mikrobielle Dosiseinheit nach Anspruch 14, in Form eines Beutels, welcher die Zusammensetzung enthält.

17. Mikrobielle Dosiseinheit nach Anspruch 14, in Form einer Tablette.

18. Mikrobielle Dosiseinheit nach Anspruch 14, in Form einer pulverförmigen Formel für Ernährungszwecke.

19. Verfahren zur Herstellung der Zusammensetzung nach einem beleibigen der Ansprüche 1 bis 13, wobei es die folgenden Schritte umfasst:

(i) Bereitstellen (a) eines getrockneten Mikroorganismus sowie (b) eines Phosphatsalzes in Pulverform; und
(ii) Vermischen des getrockneten Mikroorganismus und des Phosphatsalzes in Pulverform, um die Zusammensetzung bereitzustellen,

wobei das Phosphatsalz aus $K_2HPO_4$, $KH_2PO_4$, $Na_2HPO_4$, $NaH_2PO_4$, $MgHPO_4$, $Mg[H_2PO_4]_2$ und deren Mischungen ausgewählt ist; und wobei das Phosphatsalz in einer Menge von mindestens 50 Gew.-% vorliegt, bezogen auf das Gesamtgewicht an Mikroorganismus und Phosphatsalz.

## Revendications

1. Composition comprenant un mélange de (a) un micro-organisme séché, et (b) un sel de phosphate sous forme de poudre, le sel de phosphate étant choisi parmi $K_2HPO_4$, $KH_2PO_4$, $Na_2HPO_4$, $NaH_2PO_4$, $MgHPO_4$, $Mg[H_2PO_4]_2$ et des mélanges correspondants, et le sel de phosphate étant présent en une quantité d'au moins 50 % en poids sur la base du poids total de micro-organisme et de sel de phosphate.

2. Composition selon la revendication 1, le sel de phosphate étant choisi parmi $K_2HPO_4$, $KH_2PO_4$, $Na_2HPO_4$, $NaH_2PO_4$ et des mélanges correspondants.

3. Composition selon la revendication 1, le sel de phosphate étant choisi parmi $K_2HPO_4$, $KH_2PO_4$ et des mélanges correspondants.

4. Composition selon la revendication 1, 2 ou 3, le sel de phosphate étant un mélange de sels de $HPO_4^{2-}$ et $H_2PO_4^{-}$.

**5.** Composition selon une quelconque revendication précédente, le sel de phosphate étant ajusté en pH à un pH compris entre pH 6 et 9.

**6.** Composition selon l'une quelconque des revendications précédentes, le sel de phosphate étant ajusté en pH à un pH compris entre 6,8 et 7,2.

**7.** Composition selon une quelconque revendication précédente, le sel de phosphate étant présent en une quantité d'au moins 60 % en poids sur la base du poids total de micro-organisme et de sel de phosphate.

**8.** Composition selon une quelconque revendication précédente, le sel de phosphate étant présent en une quantité d'au moins 80 % en poids sur la base du poids total de micro-organisme et de sel de phosphate.

**9.** Composition selon une quelconque revendication précédente, le micro-organisme étant lyophilisé.

**10.** Composition selon une quelconque revendication précédente, le micro-organisme étant un probiotique.

**11.** Composition selon une quelconque revendication précédente, le micro-organisme étant choisi parmi des lactobacilli, des bifidobactéries, des saccharomyces et des mélanges correspondants.

**12.** Composition selon une quelconque revendication précédente, le micro-organisme étant choisi parmi *Bacillus coagulans, Bifidobacterium longum subsp. infantis, Lactobacillus acidophilus, Lactobacillus paracasei, Lactobacillus johnsonii, Lactobacillus plantarum, Lactobacillus reuteri, Lactobacillus reuteri protectis, Lactobacillus reuteri prodentis, Saccharomyces boulardii, Lactobacillus rhamnosus, Lactobacillus casei, Lactobacillus plantarum, Lactobacillus paracasei* et des mélanges correspondants.

**13.** Composition selon une quelconque revendication précédente, le micro-organisme étant un *Lactobacillus acidophilus.*

**14.** Dose microbienne unitaire comprenant une composition selon l'une quelconque des revendications 1 à 13.

**15.** Dose microbienne unitaire selon la revendication 14, sous la forme d'une capsule contenant la composition.

**16.** Dose microbienne unitaire selon la revendication 14, sous la forme d'un sachet contenant la composition.

**17.** Dose microbienne unitaire selon la revendication 14, sous la forme d'un comprimé.

**18.** Dose microbienne unitaire selon la revendication 14, sous la forme d'une formule nutritionnelle pulvérulente.

**19.** Procédé pour la préparation de la composition selon l'une quelconque des revendications 1 à 13, comprenant les étapes de :

(i) fourniture (a) d'un micro-organisme séché, et (b) d'un sel de phosphate sous forme de poudre ; et
(ii) mélange du micro-organisme séché et du sel de phosphate sous forme de poudre, pour fournir la composition,

le sel de phosphate étant choisi parmi $K_2HPO_4$, $KH_2PO_4$, $Na_2HPO_4$, $NaH_2PO_4$, $MgHPO_4$, $Mg[H_2PO_4]_2$ et des mélanges correspondants ; et le sel de phosphate étant présent en une quantité d'au moins 50 % en poids sur la base du poids total de micro-organisme et de sel de phosphate.

Fig. 1.

Fig. 2.

Fig. 3.

**Impact percentage of $K_2HPO_4$ in probiotic /$K_2HPO_4$/ MCC blend with a constant probiotic concentration after 6 months in humid condition (Aw 0.4 at 30°C)**

Fig. 4.

EP 3 571 287 B1

Fig. 5.

Impact excipient pH on the freeze dry Lactobacillus acidophilus powder stability in humid condition (Aw 0.4) at 30°C after 3 months

Fig. 6.